# EUROPEAN PATENT SPECIFICATION

(11) **EP 1 377 278 B1**
(45) Date of publication and mention of the grant of the patent: **02.11.2006**
(21) Application number: 02713070.7
(22) Date of filing: 10.04.2002
(51) Int. Cl.: A61K 31/40, A61K 31/497, A61P 3/10

(54) **TREATMENT OF TYPE 2 DIABETES WITH INHIBITORS OF DIPEPTIDYL PEPTIDASE IV**
BEHANDLUNG VON TYP II DIABETES MIT DIPEPTIDYL-PEPTIDASE-IV-HEMMERN
TRAITEMENT DU DIABETE DE TYPE 2 A L'AIDE D'INHIBITEURS DE DIPEPTIDYLPEPTIDASE IV

(30) Priority: 11.04.2001 GB 0109146
(43) Date of publication of application: 07.01.2004
(73) Proprietor: Ferring BV, 2132 JX Hoofddorp (NL)
(72) Inventor: BROQUA, Pierre, Ferring Research Institute, Southampton SO16 7NP (GB); SUDRE, Beatrice, Fondation pour Recher. Medicales, 1205 Geneva (CH); AUBERT, Michel, L, 1211 Geneva 14 (CH)
(74) Representative: Bates, Philip Ian
(86) International application number: PCT/GB2002/001674
(87) International publication number: WO 2002/083109

(56) References cited:
- WO-A-00/47219
- WO-A-00/56296
- WO-A-01/34594
- WO-A-01/40180
- WO-A-01/72290
- WO-A-01/81337
- WO-A-01/97808
- WO-A-95/15309
- WO-A-97/40832
- WO-A-99/61431
- DE-U- 29 909 208
- US-A- 6 107 317
- US-A- 6 110 949
- US-A- 6 124 305
- US-A- 6 166 063
- HOLST J J ET AL: "INHIBITION OF THE ACTIVITY OF DIPEPTIDYL-PEPTIDASE IV AS A TREATMENT FOR TYPE 2 DIABETES" DIABETES, NEW YORK, NY, US, vol. 47, November 1998 (1998-11), pages 1663-1670, XP000853619 ISSN: 0012-1797
- FREYSE E J ET AL: "Long-term metabolic effect of daily oral application of DPIV-inhibitor P32/98 in diabetic Zucker rats (fa/fa)." DIABETOLOGIA, vol. 43, no. Supplement 1, August 2000 (2000-08), page A67 XP008005148 36th Annual Meeting of the European Association for the Study of Diabetes;Jerusalem, Israel; September 17-21, 2000 ISSN: 0012-186X
- BRAND CHRISTIAN L ET AL: "Chronic administration of valine pyrrolidide, a selective inhibitor of dipeptidyl peptidase IV, improves glucose tolerance without affecting food intake in Zucker Obese rats." DIABETES, vol. 48, no. SUPPL. 1, 1999, page A271 XP008005144 59th Scientific Sessions of the American Diabetes Association;San Diego, California, USA; June 19-22, 1999 ISSN: 0012-1797
- BALKAN B ET AL: "Sustained improvement of glucose tolerance by DPP-IV inhibition after chronic treatment with NVP-DPP728." DIABETOLOGIA, vol. 42, no. SUPPL. 1, August 1999 (1999-08), page A41 XP008005150 35th Annual Meeting of the European Association for the Study of Diabetes;Brussels, Belgium; September 28-October 2, 1999 ISSN: 0012-186X

## Description

### FIELD OF THE INVENTION

The present invention relates to the use of dipeptidyl peptidase IV inhibitors for the manufacture of a medicament for delaying the onset of type 2 diabetes and alleviating the physiological consequences of type 2 diabetes.

### BACKGROUND

Diabetes mellitus affects about 5% of the population, and type 2 diabetes, also known as non-insulin dependent diabetes mellitus, accounts for more than 80% of all cases. Type 2 diabetes is particularly prevalent in obese people aged over 40. Complications of type 2 diabetes include retinopathy and nephropathy, and diabetics have a significantly increased chance of suffering cardiovascular disease.

A number of drugs are available for the treatment of type 2 diabetes, but new ones, particularly those acting by novel mechanisms, are still needed. One such class of candidate therapeutic agents comprises inhibitors of dipeptidyl peptidase IV (DP-IV, EC.3.4.14.5). These compounds act, at least in part, by blocking the inactivation of endogenous incretins such as GLP-1 and GIP, resulting in an increased sensitivity to insulin and reduced post-prandial hyperglycaemia. To date, however, these compounds have only been examined as a method for controlling the management of blood glucose levels on an acute basis. The implications of long-term treatment with these compounds have not been considered.

WO-A-0047219 discloses the use of a PYY therapeutic, which can be a DPPIV inhibitor, for the treatment of glucose metabolic disorders.

Holst et al. postulate in Diabetes, 47, 1663-1670, 1998 that DPP-IV inhibitors may prevent of delay type 2 diabetes and its complications.

The post-published application WO-A-0181337 discloses the treatment of, *inter alia,* impaired glucose tolerance or type 2 diabetes with DPPIV inhibitors.

The post-published application WO-A-0197808 discloses the use of a combination of a DPPIV inhibitor and an antidiabetic agent for the treatment of Type 2 diabetes and conditions associated with diabetes.

### SUMMARY OF THE INVENTION

We have now found that chronic treatment with inhibitors of DP-IV in a standard animal model of type 2 diabetes results in a delay in the development of the disease.

### DETAILED DESCRIPTION OF THE INVENTION

We have examined the effects of chronic treatment of Zucker Diabetic Fatty (ZDF) rats with inhibitors of DP-IV. The ZDF rat is a well known model for human type 2 diabetes. ZDF rats are hyperphagic, and when fed on a high fat diet they become diabetic, as shown by hyperglycaemia, hypertrigyceridaemia, polydipsia and an increase in circulating free fatty acids. Disease onset is observed at about 8 weeks and the animals are fully diabetic by 11 weeks of age. We found that chronic treatment of ZDF rats with inhibitors of DP-IV leads to a significant delay in the onset of the diabetic state, which indicates that such chronic treatment will be useful in human subjects at risk of developing type 2 diabetes, or in the early stages of the disease.

Accordingly, in a first aspect of the present invention there is provided the use, in the preparation of a pharmaceutical composition which is a depot formulation for treating an individual at risk of developing type 2 diabetes so as to delay the onset of the disease, or for treating an individual with early stage type 2 diabetes to delay the progression of diabetic complications, of an inhibitor of dipeptidyl peptidase IV, which is selected from (2S)-1-((2'S)-2'-amino-3',3'-dimethylbutanoyl)pyrrolidine-2-carbonitrile or a pharmaceutically active salt thereof: or (2S)-1-((2'S)-2'-amino-5'-pyrazinecarbonylamino pentanoyl)pyrrolidine-2-carbonitrile or a pharmaceutically active salt thereof.

The assessment that an individual is at risk of developing type 2 diabetes will generally be made by an experienced physician, who will take into consideration such factors as the age and weight (and more specifically the body mass index, BMI) of the individual, as well as any history of diabetes in the individual's family and other risk factors.

Suitable inhibitors of DP-IV should preferably be selective, i.e. they should not significantly inhibit other unrelated enzymes at a concentration equal to that at which they inhibit DP-IV, and more preferably they should not inhibit such enzymes at a concentration ten-fold greater, even more preferably one hundred-fold greater, than that at which they significantly inhibit DP-IV.

The compounds have at least one basic nitrogen atom and so are able to form addition salts with protic acids. Examples of such acids include hydrochloric acid, sulphuric acid, phosphoric acid, acetic acid, trifluoroacetic acid, fumaric acid, maleic acid, citric acid, benzoic acid, pamoic acid and methanesulphonic acid. Insofar as these acids are pharmaceutically acceptable, such salts are included within the scope of the present invention.

The formulation containing the inhibitor of DP-IV may further include one or more additional pharmacologically active agents, such as anti-diabetic agents, but preferably the DP-IV inhibitor is the sole active agent.

The formulation is to be administered as a depot which releases active compound over a period of between one week and three months. Such controlled-release formulations are known in the art, and generally comprise a pharmaceutically active species associated with a biocompatible power. The polymer may simply encapsulate the active agent, forming a physical barrier to its release into the general circulation, or there may be a chemical association, such as a covalent or ionic interaction, between the polymer and the active agent. Such formulations are administered by Intramuscular or subcutaneous injection. In this case, the administration will be repeated at intervals of one week up to three months so as to maintain treatment over an extended period.

The invention will now be described in more detail with reference to the attached drawings in which Figs. 1 to 7 illustrate results in Example 3, below.
FIGURE 1 shows blood glucose concentration;
FIGURE 2 shows plasma insulin concentration;
FIGURE 3 shows body weight gain;
FIGURE 4 shows food consumption;
FIGURE 5 shows water intake;
FIGURE 6 shows blood-free fatty acid concentration; and
FIGURE 7 shows blood triglyceride concentration.

### EXAMPLES

### Example 1 - Preparation of Inhibitors of DP-IV

Inhibitors of DP-IV can be prepared according to published methods.

### Example 1A - (2S)-1-(2'S)-2'-amino-3',3'-dimethylbutanoyl)pyrrolidine-2-carbonitrile hydrochloride

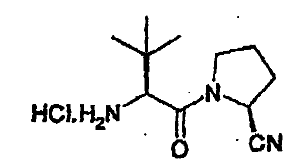

The title compound is prepared according to the methods of WO95/15309, and particularly of Example 18 therein. Briefly, BOC-protected tert-butylglycine is coupled to prolineamide, the primary amide function is dehydrated with trifluoroacetic anhydride to give the nitrile, and the BOC-group is removed with HCl in dioxan.

### Example 1B - (2S)-1-((2'S)-2'-amino-6'-pyrazinecarbonylaminopentanoyl)pyrrolidine-2-carbonitrile trifluoroacetate

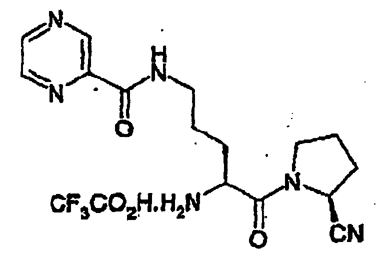

The title compound is prepared according to the method of Example 1A above. Briefly, N^{α}-BOC-protected N^{ω}-pyrazinecabonylornithine is coupled to prolineamide, the primary amide function is dehydrated with trifluoroacetic anhydride to give the nitrile, and the BOC. group is removed with trifluoroacetic acid.

### Comparative Example 1C - N-isoleucylthiazolidine hydrochloride

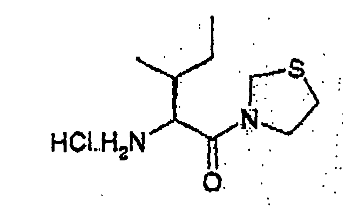

The title compound is prepared according to the standard methods. Briefly, BOC-protected isoleucine is coupled to thiazolidine and the BOC-group is removed with HCl in dioxan.

### Comparative Example 1D-(2S)-1-((2'-(5"-Cyano-2"-pyridylamino)ethylamino)acetyl)pyrrolidine-2-carbonitrile

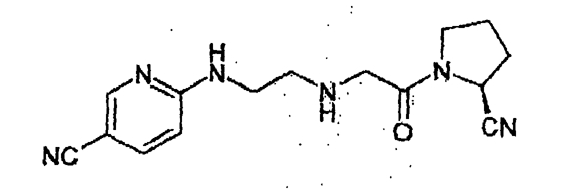

The title compound is prepared according to the methods of WO98/19998, and particularly of Example 3 therein. Briefly, bromoacetyl bromide is reacted with prolineamide and the product is dehydrated with trifluoroacetic anhydride to give N-bromoacetylpyrrolidine-2-carbonitrile. This is treated with 2-(5-cyano-2-pyridylamino)ethylamine to give the product.

### Example 2 - inhibition of DP-IV in vitro

The *in vitro* inhibitory action of the compounds is determined in a fluorimetric assay. Human DP-IV is incubated with a standard substrate, Ala-Pro-AFC, in the presence of various concentrations of the inhibitor. The reaction is monitored by measuring the increase in fluorescence due to the reaction product, AFC. Using standard manipulations, an inhibitory constant, Kᵢ, is determined. Typical results are given below.

| Compound of Example No. | Kᵢ(nM) |
|---|---|
| 1A | 1.0 |
| 1B | 0.4 |
| 1C (comparative) | 33.0 |
| 1D (Comparative) | 5.0 |

### Example 3 - Effect of long term inhibition of DP-IV in ZDF rat

Male ZDF rats, aged 6.5 weeks at the beginning of the study (day 0), are given the compound of Example 1A (10mg/kg p.o.) once or twice per day for four weeks. Control - animals are given vehicle. A group of untreated lean rats is used as a comparison. Glycaemia, insulinaemia, body weight, food and water intake, and plasma triglyceride and free fatty acid levels are monitored throughout the study.

### Example 3A- Glycaemia

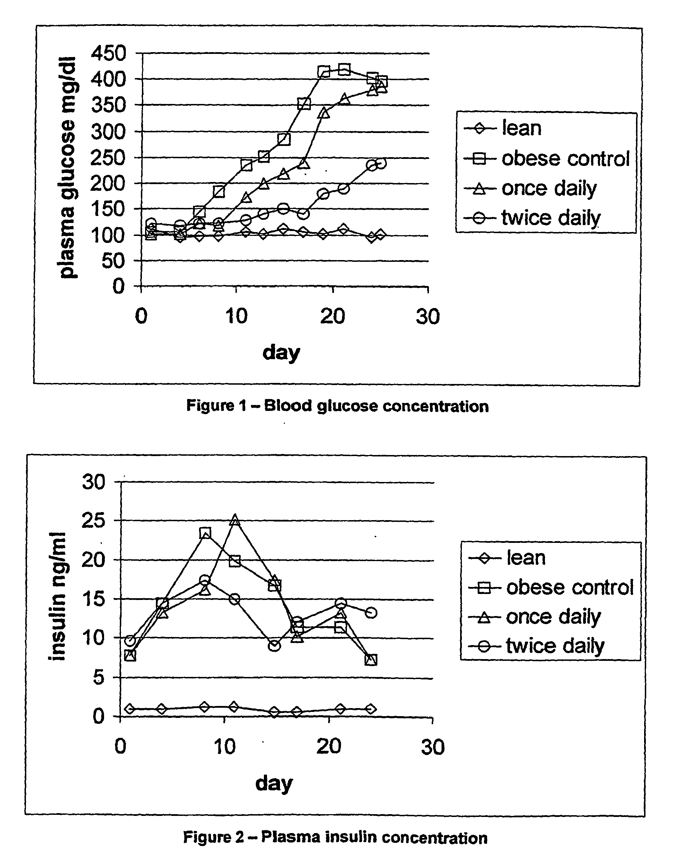

At the start of the study glycaemia is not significantly different in the obese animals compared to the lean rats. At day 8, the obese control group develop hyperglycaemia, which increases and reaches a plateau by day 19. The animals treated once daily with the inhibitor do not develop significant hyperglycaemia until day 15, and those treated twice daily do not develop significant hyperglycaemia until day 24. The results are presented in Figure 1.

### Example 3B - Insulinaemia

All three groups of obese animals show elevated plasma insulin concentrations at the beginning of the study period. In the control obese animal group, the insulin concentration rises rapidly to reach a peak by day 8 before decreasing as the islet β-cells die. In the group treated once daily with the inhibitor a similar pattern is observed, but the peak insulin level is only reached on day 11. In the group treated twice daily with the inhibitor insulin concentration does not attain the same high level, and there is evidence of β-cell survival at the end of the study period. The results are presented in Figure 2.

### Example 3C - Body weight gain

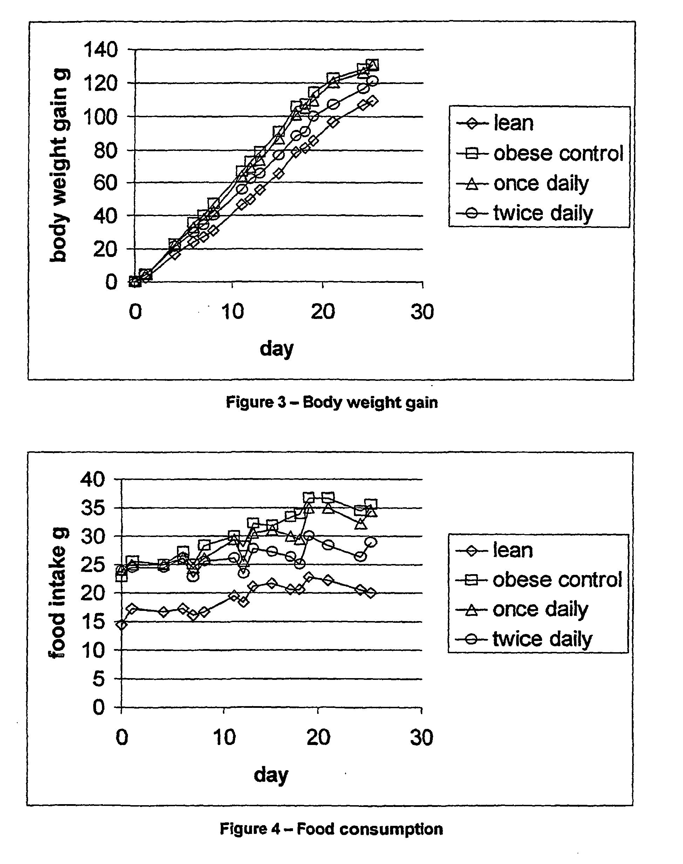

All three groups of obese animals gain weight faster than the lean group, but the group treated twice daily with the inhibitor gain less weight than the control obese group and the group treated once daily. The results are presented in Figure 3.

### Example 3D - Food and water intake

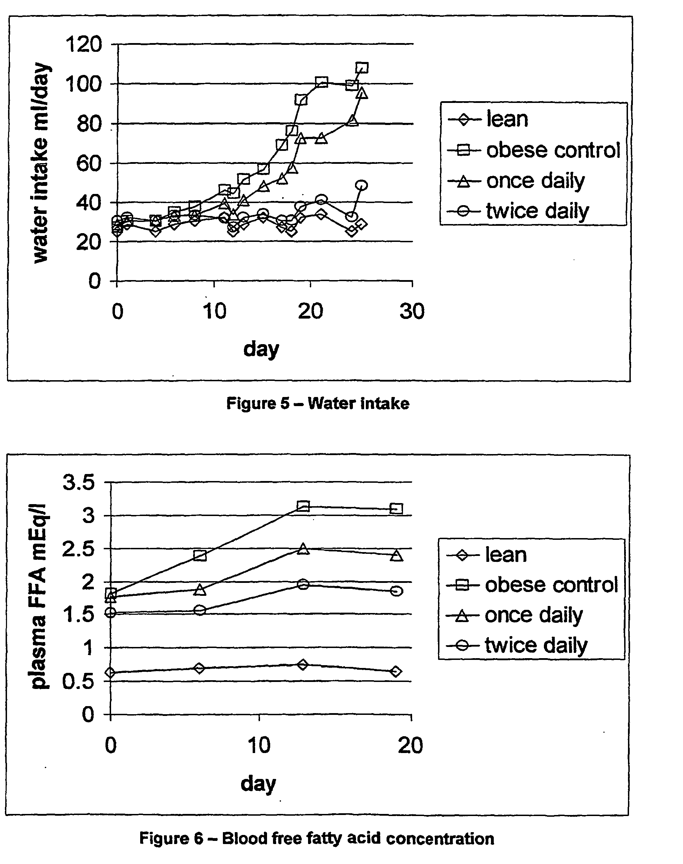

All three groups of obese animals eat more than the lean group, but from day 17 the food intake for the group treated twice daily is significantly less than that for the control obese group and the once-daily treatment group. From day 10, the control and once-daily treatment groups show an increase in their water consumption, but the twice-daily treatment group maintains a normal water intake. The results are presented in Figures 4 and 5.

### Example 3E - Plasma free fatty acid and triglyceride levels

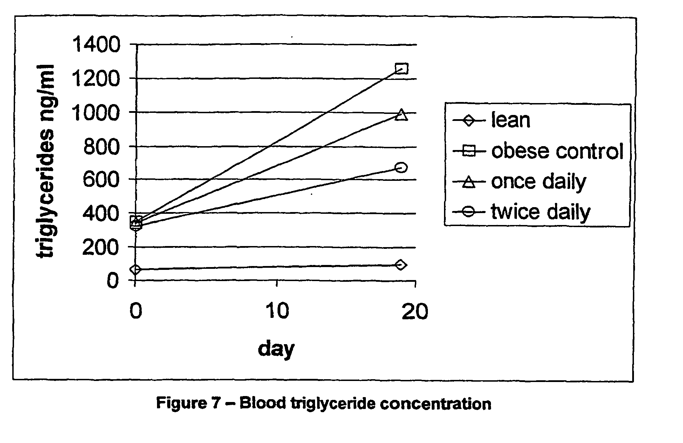

Plasma free fatty acid and triglyceride levels are significantly elevated in the obese animals at day 0, and in control obese animals they increase throughout the study period. Once-daily, and particularly twice-daily treatment attenuates this increase. The results are presented in Figures 6 and 7.

The results described above clearly indicate that long-term inhibition of DP-IV is effective in delaying the onset of diabetic symptoms in the ZDF rat, and hence that inhibitors of DP-IV should be useful as prophylactic agents for people at risk of developing type 2 diabetes and as a treatment for people in the early stages of the disease to delay the progression of diabetic complications.

## Claims

1. The use, in the preparation of a pharmaceutical composition which is a depot formulation for treating an individual at risk of developing type 2 diabetes so as to delay the onset of the disease; or for treating an individual with early stage type 2 diabetes to delay the progression of diabetic complications, of an inhibitor of dipeptidyl peptidase IV, which is selected from (2S)-1-((2'S)-2'-amino-3',3'-dimethylbutanoyl)pyrrolidine-2-carbonitrile or a pharmaceutically active salt thereof; or (2S)-1-((2'S)-2'-amino-5'-pyrazinecarbonylaminopantanoyl)pyrrolidine-2-carbonitrile or a pharmaceutically active salt thereof.

2. The use according to claim 1 in which the pharmaceutical composition releases the inhibitor of dipeptidyl peptidase IV over a period of between one week and three months.

3. The use according to claim 1 or claim 2 in which the individual is a human.

## Patentansprüche

1. Verwendung eines Inhibitors von Dipeptidylpeptidase IV, ausgewählt aus (2S)-1-((2'S)-2'-Amino-3',3'-dimethylbutanoyl)pyrrolidin-2-carbonitril oder einem pharmazeutisch aktiven Salz davon; oder (2S)-1-((2'S)-2'-Amino-5'-pyrazincarbonylaminopentanoyl)pyrrolidin-2-carbonitril oder einem pharmazeutisch aktiven Salz davon, für die Herstellung einer pharmazeutischen Zusammensetzung, die eine Depotformulierung ist, zur Behandlung eines Individuums, das für eine Erkrankung an Diabetes Typ 2 gefährdet ist, um den Ausbruch der Krankheit zu verzögern, oder zur Behandlung eines Individuums mit Diabetes Typ 2 im Frühstadium, um die Entwicklung diabetischer Komplikationen zu verzögern.

2. Verwendung nach Anspruch 1, wobei die pharmazeutische Zusammensetzung den Inhibitor von Dipeptidylpeptidase IV über einen Zeitraum zwischen einer Woche und drei Monaten freisetzt.

3. Verwendung nach Anspruch 1 oder Anspruch 2, wobei das Individuum ein Mensch ist.

## Revendications

1. Utilisation d'un inhibiteur de dipeptidyl-peptidase IV qui est sélectionné parmi le (2S)-1-((2'S)-2'-amino-3'-3'-diméthylbutanoyl)pyrrolidine-2-carbonitrile ou un sel pharmaceutiquement actif du même; ou le (2S)-1-((2'S)-2'-amino-5'-pyrazine-carbonylamino-pentanoyl)pyrrolidine-2-carbonitrile ou un sel pharmaceutiquement actif du même, dans la préparation d'une composition pharmaceutique qui est une formulation à libération prolongée pour traiter un individu à risque de développer un diabète de type 2 afin de retarder le début de la maladie, ou pour traiter un individu souffrant de diabète de type 2 de stade précoce afin de retarder la progression de complications diabétiques.

2. L'utilisation selon la revendication 1, dans laquelle la composition pharmaceutique libère l'inhibiteur de dipeptidyl-peptidase IV sur une période d'entre une semaine et trois mois.

3. L'utilisation selon la revendication 1 ou 2, dans laquelle l'individu est un humain.
